**Europäisches Patentamt**

**European Patent Office**

**Office européen des brevets**

(11) Publication number: **0 005 615**

**A1**

(12) **EUROPEAN PATENT APPLICATION**

(21) Application number: **79300812.9**

(22) Date of filing: **11.05.79**

(51) Int. Cl.²: **A 63 B 71/12**
**A 61 F 5/04**

(30) Priority: **12.05.78 US 905470**
**13.09.78 US 941946**
**03.04.79 US 26519**

(43) Date of publication of application:
**28.11.79 Bulletin 79/24**

(84) Designated Contracting States:
**DE FR GB IT**

(71) Applicant: **Rhee, Jhoon Goo**
**2000 L Street, N.W.**
**Washington, D.C. 20007(US)**

(72) Inventor: **Rhee, Jhoon Goo**
**2000 L Street, N.W.**
**Washington, D.C. 20007(US)**

(74) Representative: **Wood, John Irwin**
**45 Kent Avenue**
**Ealing London W13 8BE(GB)**

(54) A protective device for parts of the body.

㉟ A protective device for a part of a body is made up of a laminated material which has a basic layer (554) of an energy absorbing material. To this may be added a stiffening layer (552), an outer protective layer (550) and an inside soft ventilating layer (558).

The device is contoured at its edges to fit various parts of the body and is able to be secured by straps or flaps which can include self fastening elements.

Fig.26

EP 0 005 615 A1

## A protective device for parts of the body

This invention relates to a protective device for parts of the body and is able to be used to function as a medical support while curative treatment is being undertaken, or as a protective device during an event, such as a sporting event.

When an injury to a limb or other part of a body occurs such as breakage or a severe strain it is necessary in order to be able to cure the limb to hold it securely and rigid in a prescribed position. Sometimes it is necessary for the limb to be held temporarily such as when a patient is being removed to hospital and in such cases splint and bandage arrangements are usually used. These are useful as an immediate treatment but can be uncomfortable for the wearer and are not necessarily convenient to carry and to apply at the scene of an accident. It is therefore a requirement that a readily applicable easily portable protective device which gives adequate and comfortable support be available and the present invention has for an object to provide such a device.

Similarly in hospitals and the like surgeons will set limbs and strained parts of the body in casts which conventionally are made from plaster-of-paris. This sets rigid, is messy to apply, and is often uncomfortable for the patient since it is very heavy and once applied cannot readily be removed. If it is applied so as to cause discomfort a surgeon is reluctant to remove it since it has to be destroyed and a new cast made. Also the injured part cannot be examined at will.

It is another object of the invention to provide a protective device which gives the same degree of support as a plaster cast but which is more easily applied and which can be readily removed and replaced and which also is light and comfortable for the wearer.

In many sporting activities, particularly those involving hard balls, the players find it necessary to protect vulnerable parts of their body from damage which might occur from hard knocks. They require a protective device which is light and comfortable and which is also able to be removed at will. It is a further object of the invention to provide such a device.

According to the present invention a protective device for parts of the body comprises a sheet of energy absorbing cellular material having a contoured edge, the sheet being bendable to fit around a selected part of the body, and fastening means for securing the device around the selected part.

By being of an energy absorbing material such as a closed cell plastics material the device offers the advantage of protection against shock from knocks. This can be enhanced by including a laminated layer of a stiffening material. An outer protective layer may further be added to enable the device to be kept clean, dry and undamaged by accidental knocks.

In order that the wearer's skin may be properly ventilated an open cell layer of soft material can with advantage be placed next to the skin and ventilation holes may be formed through all the layers.

For sporting applications the outer layer may be constructed from a cellular material, preferably of a closed cell material. In order to fit around a part of a body better the energy absorbing cellular material
may be selected as a material having virtually no
memory.

To permit easy and ready removal of the device straps
can be formed on, or secured around, the device.
The straps may have their own buckles or may also, or
alternatively, be secured with self fastening means
such as hook and pile fasteners.

The sheet-like protective cast material has several
other advantages, among which are the following.
When formed into cylinders the inner walls do not
uniformly contact all skin surfaces, providing
ventilation over the skin. The devices can be
economically produced, shipped and stored. One device
is adjustable and useful to fit a wide range of
varied sizes. It is also able to be reused.

The devices are able to be used with other materials
where necessary, such as wood so that a foot support
is able to be constructed and the layers of the device
nailed to the sole member. The sole member may then
be of a durable material and fitted with a non-shine
surface to ensure easy walking. Alternatively it may
be preferable to secure the device over and above a
normal shoe.

The device is referred to hereinafter as a cast in order
that it may be equated in the reader's mind with medical
applications, although it is to be appreciated it is
not limited to such applications.

When the material is bent in one direction it gives
the advantage that it will resist bending in another
direction.

The present invention and various embodiments thereof will now be described by way of example with reference to the various embodiments as shown in the accompanying drawings, in which

Figure 1 is an outer planar view of a first embodiment of the protective device of the invention to be worn around the ankle and foot;

Figure 2 is a right side perspective view of the device of Figure 1 shown as worn around the right foot;

Figure 3 is a left side perspective view of the device as shown in Figure 2;

Figure 4 is a view, partly in section, taken along line 4-4 of Figure 1;

Figure 5 is an outer planar view of a second embodiment of the protective device of the invention to be worn around the foot and ankle usable with a shoe when worn on the foot;

Figure 6 is a left side perspective view of the device of Figure 5 shown as worn on the right foot;

Figure 7 is a right side perspective view of the device as shown in Figure 6;

Figure 8 is a perspective top and front view of the device shown in Figure 6;

Figure 9 is an outer planar view of a third embodiment of a protective device of the invention to be worn on the back of the hand and round the thumb or around two adjacent fingers of the hand;

Figure 10 is a perspective palm view of the device of Figure 9 as worn around the thumb and back of the right hand;

Figure 11 is a perspective palm view of the device of Figure 9 as worn around the index and adjacent fingers of the right hand;

Figure 12 is a perspective palm view of the device of Figure 9 as worn round the little and adjacent fingers of the right hand;

Figure 13 is an outer planar view of a fourth embodiment of the protective device of the invention to be worn around the hand and wrist;

Figure 14 is an inner planar view of the protective device of Figure 13;

Figure 15 is a perspective side view of the device of Figure 13 as worn around the left hand and wrist;

Figure 16 is a cross sectional view taken along line 16-16 of Figure 14;

Figure 17 is an outer planar view of a fifth embodiment of the protective device of the invention to be worn around the knee and adjacent leg portions or around the elbow and adjacent arm portions;

Figure 18 is an inner planar view of the protective device of Figure 17.

Figure 19 is a perspective side view of the device of Figure 17 as worn around a person's knee or elbow and adjacent leg or arm portions;

Figure 20 is a cross-sectional view taken along line 20-20 of Figure 18 showing the basic laminated construction and a modification of the construction of the sandwich-like material of the invention;

Figure 21 is an outer planar view of a sixth embodiment of the protective device of the invention to be worn around the neck and back of the head;

Figure 22 is a perspective side view of the device of Figure 21 as worn around a person's neck and back of the head;

Figure 23 is a cross-sectional view taken along line 23-23 of Figure 21, showing details of the chin-supporting member;

Figure 24 shows a modified chin-supporting member of the device of Figure 21;

Figure 25 is a cross-sectional view taken along line 25-25 of each of the devices shown in Figures 1, 5, 9, 13, 17, 21 and 27 showing the basic laminated construction of the sandwich-like material of the invention;

Figure 26 is a cross-section taken along line 26-26 of each of the devices shown in Figures 1, 5, 9, 13, 17, 21 and 27 illustrating a modification of the basic laminated construction of the sandwich-like material shown in Figure 25;

Figure 27 is an outer planar view of a seventh embodiment of the protective device of the invention to be worn around a person's midsection to protect the back, rib cage and abdomen;

Figure 28 is a perspective front view of the device of Figure 27 as worn by a person; and

Figure 29 is a perspective back view of the device of Figure 27 as worn by a person.

Figure 30 shows a preferred form of a knee cast.

Figure 31 shows the inside of the knee cast shown in Figure 30.

Figure 32 shows an elbow cast.

Figure 33 shows the rear view of the elbow cast in Figure 32.

Figure 34 shows a preferred form of an ankle cast.

Figure 35 is a detail of the ankle cast shown in Figure 34.

Figure 36 shows one form of a wrist and hand cast.

Figure 37 shows another form of a wrist and hand cast.

Figure 38 shows a modification of the wrist and hand cast.

Figure 39 shows another modification of the wrist and hand cast.

Figure 40 shows a thumb support.

Figure 41 shows a modified thumb support having an exposed palm area for use in sports.

Figure 42 shows one preferred construction of the cast material.

Figure 43 shows another construction of the cast material.

Figure 44 shows an alternate preferred construction of the cast material.

Figure 45 shows one preferred construction of the cast material in which edges of the stiffening member are recessed from edges of the foam.

Figure 46 shows an embodiment of the cast material which is preferred for sports with foam on both sides of the stiffening member.

Figure 47 shows a preferred light weight embodiment of the cast material with an outer expanded layer and an inner open cell comfort and ventilation layer and an outer tough thin flexible leather-like coating.

Figure 48 shows a modification of the Figure 47 embodiment without the tough outer leather-like layer.

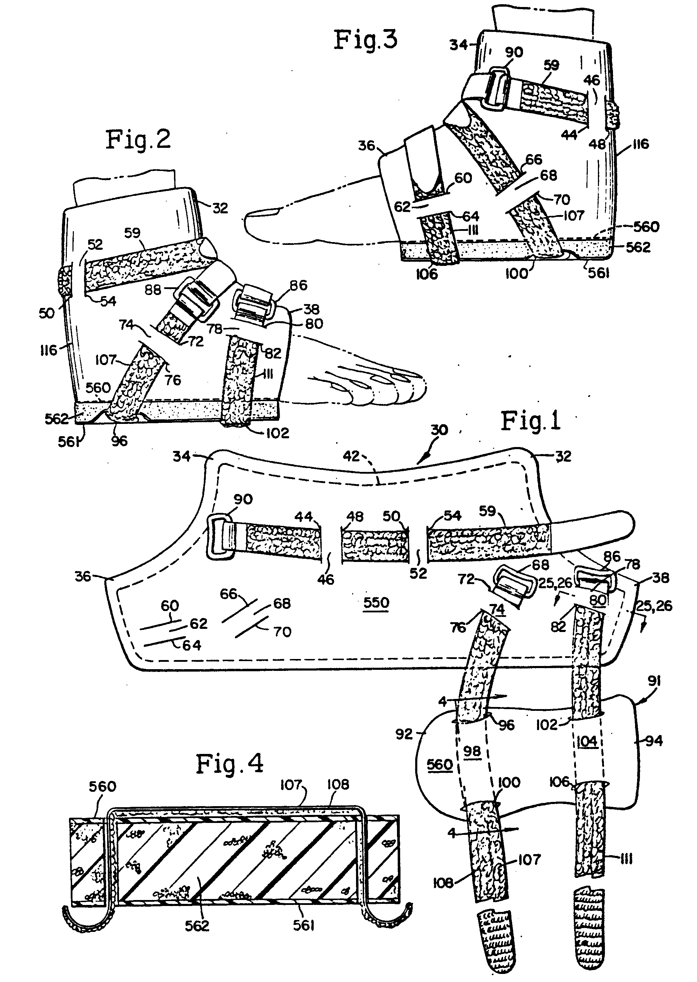

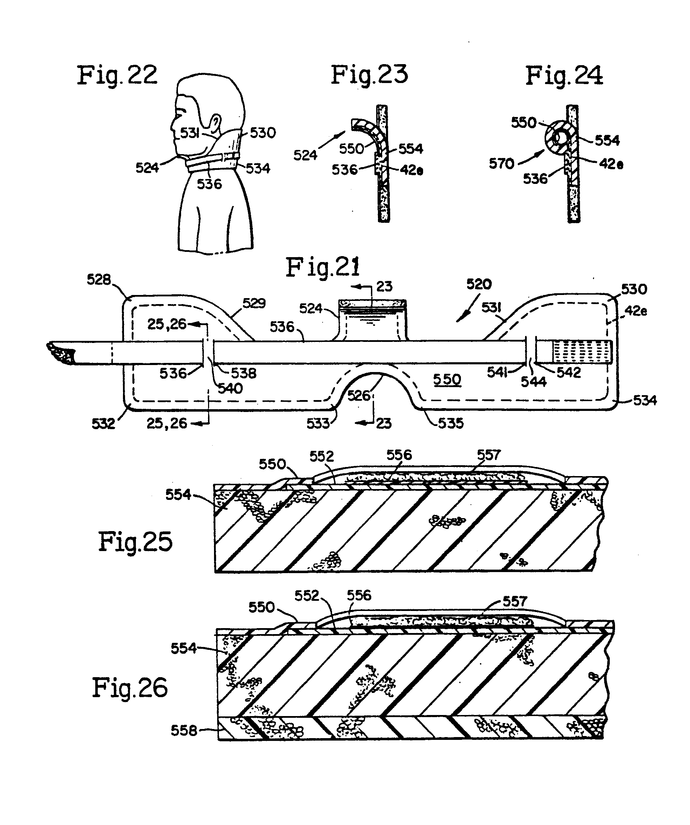

Referring now to the drawings and particularly to the first embodiment shown in Figures 1-4, this provides protection and support for the foot and ankle, and its parts are generally designated by the numerals 30 and 91. Member 30 comprises one part of the device having the sandwich-like construction of the invention which is later described with reference to Figures 25 and 26, i.e. an outer covering layer 550, a resilient, energy-absorbing layer 554, and a bendable stiffening material in layer 552 (not shown in Figures 1 to 4 but shown in Figures 25 and 26), wherein said bendable stiffening material is interposed between layers 550 and 554, with its edges inwardly displaced from the mating edges of layers 550 and 554. As shown in Figure 4, member 91 also comprises a similar sandwich-like, laminated construction of a resilient, energy-absorbing material in layer 562 interposed between an outer covering material in layer 560 and an inner covering material in layer 561, both similar to layer 550, which cover the interposed energy-absorbing material. The energy absorbing material in layer 562 comprises a closed-cell plastic foam material similar to energy-absorbing material in layer 554 of member 30.   Outer covering material 550 and 560 of members 30 and 91 is a flexible, tough, pliable material which has the overall contoured shape of member 30 and member 91 respectively.

Member 30 has a generally rectangular, contoured shape and comprises contoured portions 32, 34, 36 and 38; slits 44, 48, 50 and 54 which form loop members 46 and 52 for retaining strap member 59, slits 60, 64, 78 and 82 which forms loop members 62 and 80 for retaining strap member 111;  and slits 66, 70, 72 and 76 which form loop members 68 and 74 for retaining strap member 107. Member 91 also has a generally rectangular, contoured shape and comprises contoured portions 92 and 94; slits 96 and 100 which form loop member 98 for retaining strap member 107 with buckle 88;  and slits 102 and 106 which form loop member 104 for retaining strap member 111 with buckle 86.   Strap members 107 and 111 both contain a self fastening material 107 such as that sold under the Trade Name 'Velcro' which have hook and pile components.

The bendable stiffening member of member 30 has the same overall rectangular, contoured shape of member 30 but is slightly smaller in overall size.  The resilient, energy-absorbing material in layer 554 has the overall contoured shape of member 30 and is a flexible, energy-absorbing material, as herein described.   The resilient, energy-absorbing material in layer 562 of member 91 has the overall contoured shape of member 91 and is a flexible, energy-absorbing material, as herein described.

Figure 1 shows the peripheral edge 42 of the stiffening member wherein the peripheral edge of the stiffening member is inwardly displaced from the peripheral mating

edges of plastic foam material in layer 554 and outer covering material in layer 550. Stiffening material is retained in its inwardly displaced position by securing the mating edges of plastic foam material in layer 554 and outer covering material in layer 550 by suitable means, such as glueing, heat sealing, stitching and the like.

Retaining strap member 59 is threaded through retaining loop members 46 and 52, retaining strap member 107 is threaded through retaining loop members 74 of member 30 and 98 of member 91 and retaining strap member 111 is similarly threaded through retaining loop members 80 and 104.

Figures 2 and 3 show the first embodiment which provides protection and support to the right foot and ankle. Member 30 is wrapped and bent around the foot and ankle so as to form a relatively rigid, generally cylindrical configuration 116 around the foot and ankle. The device is placed around the foot and ankle so that contoured sections 32 and 34 abut in front of the shin of the leg, and contoured sections 36 and 38 abut at the top of the foot. Member 91 is placed on the bottom of the foot with contoured portion 92 abuting at the heel of the foot. The device is retained on the foot and ankle of the wearer by retaining strap member 59 passing through and around buckle 90, retaining strap member 107 passing through and around buckle 88, and retaining strap member 111 and passing through and around buckle 86, and each strap member end is secured by the self fastening material on the surface of the straps. As worn, member 91 forms a protective surface for the bottom heel portion of the foot. As worn and tightly secured by the strap members, members 30 and 91 retain the foot and ankle in a substantially immobilized state and restrict sideways movement of the ankle, but permit a forward bending movement of the foot and ankle by proper adjustment of the retaining strap members.

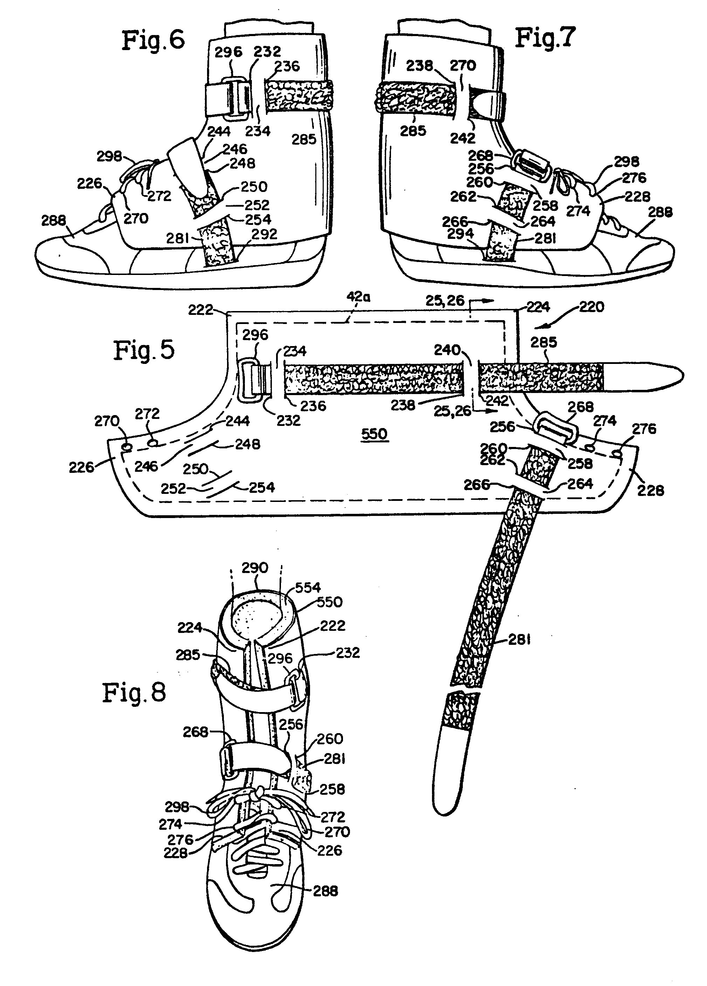

A second embodiment of the invention for the foot and ankle is shown in Figures 5-8 and is similar to the device shown in Figures 1-4. However, this embodiment is designed to be worn with a shoe on the foot to protect and support the foot and ankle. The second embodiment is generally designated by the numeral 220 and has a contoured, generally rectangular shape having the sandwich-like construction of the invention, i.e. an outer layer 550, resilient, energy-absorbing layer 554, a stiffening layer and retaining strap members 281 and 285.

The outer covering material of layer 550 is the flexible, tough, pliable material of the construction of the invention, which has the overall rectangular contoured shape of member 220. Member 220 comprises contoured portions 222, 224, 226 and 228, slits 232, 236, 238 and 242 which form loop members 234 and 240 respectively for retaining strap member 285 with buckle 296; and slits 256, 260, 262, 266, 254, 250, 248, 244 which form loop members 258, 264, 252 and 246 respectively for retaining strap member 281 with buckle 268.

A bendable stiffening material 42a in layer 552, as shown in Figure 5, has the same overall contoured shape of the device but is slightly smaller in its peripheral size. The resilient, energy-absorbing material 554 has the overall contoured shape of member 220 and is the flexible, energy-absorbing material of the invention.

The embodiment of Figures 5-8 having the resilient plastic foam layer 554, bendable stiffening layer and outer covering layer 550 are formed so as to give the sandwich-like construction of member 220. The peripheral edges of bendable stiffening material 42a is inwardly displaced from the peripheral mating edges of plastic foam layer 554 and outer covering layer 550, and bendable stiffening layer is retained in its inwardly displaced position by securing the mating edges of layers 554 and 550 by suitable means, such as glueing, heat sealing, stitching, and the like.

Figures 6, 7 and 8 show the second embodiment as worn on the right foot, wherein member 220 is bent around the foot and ankle and over a shoe 288 to form a rigid, generally cylindrical surface 290. The device is positioned so that contoured portions 222, 224, abut at the shin of the leg and contoured portions 226 and 228 abut at the top of the foot and shoe, as particularly shown in Figure 8, and by means of strap members 281 and 285, the device is retained around and on the foot, shoe and ankle. The retaining strap member 285 is placed through and around buckle 296, retaining strap member 281 is threaded through slits 292 and 294 of shoe 288, and placed through and around buckle 268. The ends of retaining strap members are secured by the self fastening material on the straps as described herein. A lace 298 is threaded through holes 270, 272, 274 and 276, as shown in Figure 8, and tied so as to aid in securing the device around and on the foot, shoe and ankle.

Member 220 forms a rigid, generally cylindrical surface 290, whereby the wearer's foot and ankle are protected and supported by the device. As worn, the ankle is retained in a substantially immobilized state, wherein sideways movement is generally restricted but wherein a forward bending movement of the ankle and foot is possible by proper adjustment of the restraining strap members.

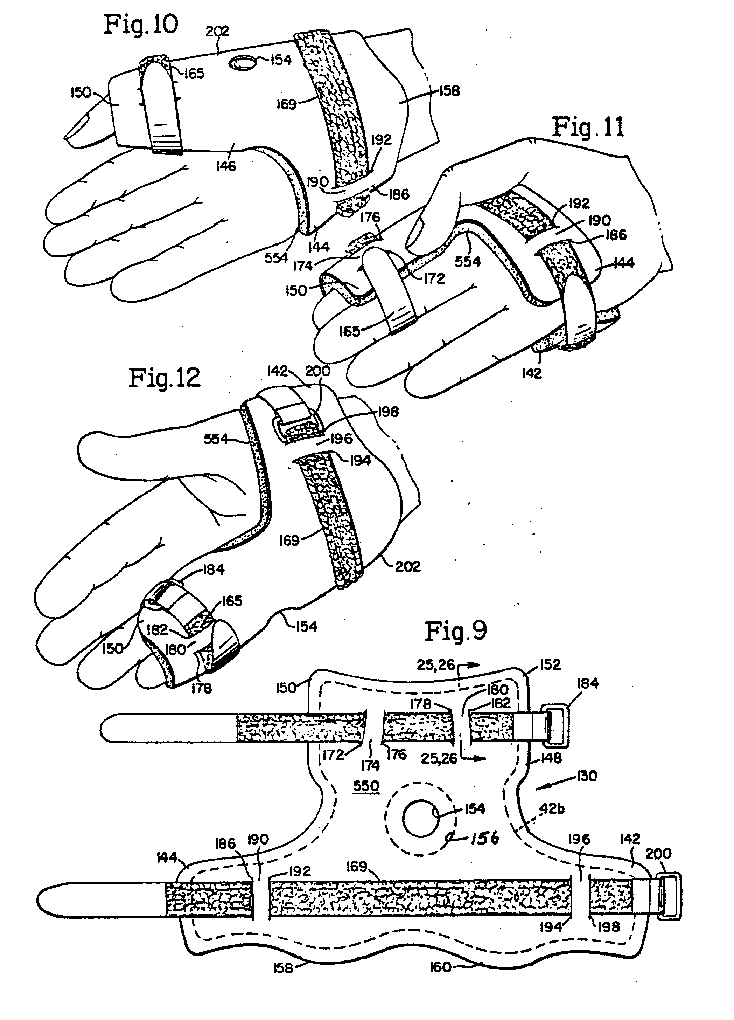

A third embodiment of the invention, shown in Figures 9-12 and generally denoted by the numeral 130, provides protection and support to the hand and fingers or thumb of the wearer, and comprises an outer covering layer 550, stiffening layer, inner, and energy-absorbing layer 554. Device 130 includes a ventilation opening 154, contoured portions 142, 144, 150, 152, slits 172, 176, 178 and 182 which form loop members 174 and 180 respectively for a retaining strap member 165 having buckle 184, and slits 186, 192, 194 and 198 which form loop members 190 and 196 respectively for a retaining strap member 169 having buckle 200.

Figure 9 shows the third embodiment of the invention with resilient layer 554, bendable stiffening material 42b and outer layer 550 formed so as to give the sandwich-like construction of member 130. The peripheral edge of stiffening material 42b is inwardly displaced from the peripheral edges of layers 554 and the edges of 550 which are secured together by suitable means as stated above. The bendable stiffening material 42b is inwardly displaced from the outer edge of the ventilation hole 154 as designated by numeral 156 in Figure 9, and is secured in said inwardly displaced position by suitable means as described above for securing the peripheral edges of layers 554 and 550. Retaining strap member 165 is shown threaded under retaining loop members 174 and 180, and retaining strap member 169 is shown threaded under retaining loop members 190 and 196.

Figure 10 shows one manner in which the device of Figure 9, can be worn around the thumb and wrist of the right hand, whereby member 130 is bent around to form a rigid, generally cylindrical surface 202 about the thumb and also the upper part of the hand and a part of the wrist. The device is positioned so that contoured portion 152 (not shown in Figure 10), and contoured portion 150 extend about the thumb and contoured portion 142 (not shown in Figure 10) and contoured portion 144 extend about and abut the wrist in the region adjacent the outer edge of the hand. The device is secured around and on the wearer's hand and thumb by retaining strap members 165 and 169 and buckles 134 and 200. The retaining strap members are secured by the self fastening material, as described above.

Figure 11 shows another manner in which the device of Figure 9, can be worn on the index and adjacent finger of the right hand. Figure 11 shows member 130 bent around so as to form a rigid, generally cylindrical surface about the index and adjacent finger of the right hand and also around the upper part of the hand and a part of the wrist. The device is positioned so that contoured portion 152 (not shown in Figure 11),and contoured portion 150 extend about the index and adjacent finger and contoured portions 142 and 144 extend about the hand in front of the thumb so that they abut the hand at its outer edge. The thumb abuts the device between contoured portions 158 and 160 (not shown in Figure 11). The device is secured on the wearer's hand and fingers by retaining strap members 165 and 169, and buckles 184 and 200. An additional retaining strap member (not shown) may also be added so as to secure the device as worn in Figure 11. This additional retaining strap member could be fastened to contoured portions 158 and 160 so that when the device is worn the retaining strap member would be placed behind the thumb so as to secure the device more firmly. The retaining straps can be secured by self fastening material, as described above.

Figure 12 shows another manner in which the device of Figure 9 can be worn on the little and adjacent finger of the right hand. The device is positioned on the hand so that contoured portion 152 (not shown in Figure 12) and contoured portion 150 extend about the little and adjacent fingers and contoured portion 144 (not shown in Figure 12) and contoured portion 142 extend about the hand so that they abut the hand near the base of the thumb. The device is secured on the wearer's hand and outer two fingers by retaining strap members 165 and 169 and buckles 184 and 200. The retaining straps are secured by the self fastening material.

Figures 10, 11 and 12 show the device of Figure 9 wherein member 130 is bent around to form a rigid, generally cylindrical surface 202 about the thumb or fingers of the hand and adjacent hand and wrist portions. The generally cylindrical surface 202 is formed as the device is bent to encircle the thumb or fingers and the hand, wherein the rigid, generally cylindrical shape is formed and retained by the bendable stiffening material 42b. The device is also adaptable for use on the left hand.

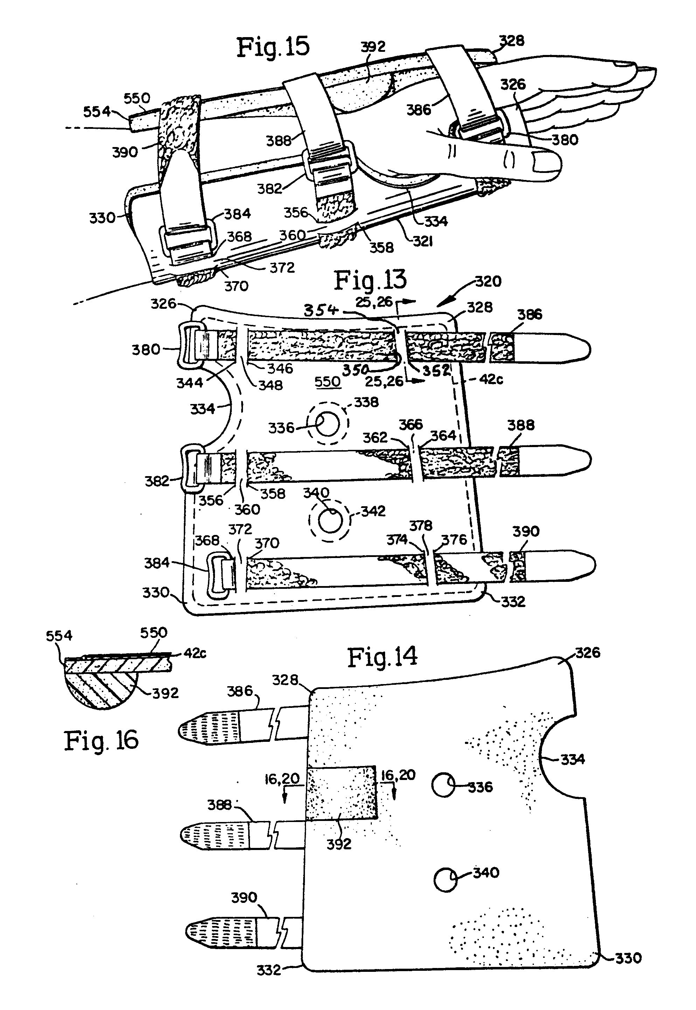

A fourth embodiment of the invention is shown in Figures 13-16 wherein the device, generally designated by the numeral 320, provides protection and support to the wrist and hand of the wearer. Member 320 comprises a contoured, generally rectangular shape having the sandwich-like construction of the invention. Member 320 also comprises retaining strap members 386, 388 and 390 with buckles 380, 382 and 384, respectively, and ventilation openings 336 and 340.

The outer covering layer 550 and layer 554 have the overall rectangular contoured shape of member 320 and comprise contoured portions 326, 328, 330, 332 and 334, slits 344, 346, 350 and 352 which form loop members 348 and 354 for retaining strap member 386, slits 356, 358, 362 and 364 which form loop members 360 and 366 respectively for retaining strap member 388 and slits 368, 370, 374 and 376 which form loop members 372 and 378 for retaining strap member 390.

The bendable stiffening material 42c has the overall contoured shape of the device but is slightly smaller in size, as shown in Figure 13. Figure 13 shows the peripheral edges of stiffening member 42c as inwardly displaced from the peripheral mating edges of layers 554 and 550. In addition, stiffening material 42c is inwardly displaced from the edge of holes 336 and 340, as shown in Figure 13 by dotted lines 338 and 342. Stiffening material 42c is retained in its interposed position by securing the mating edges of layers 554 and 550 by suitable means discussed above.

Figure 14 shows the inner surface of the device of Figure 13. As shown in Figure 14, the inner surface of layer 554 has the same overall rectangular shape of the device. The inner surface comprises either an open cell plastic foam material or a closed cell plastic foam material. In addition, the inner surface has a retaining, semi-cylindrical contouring member 392 disposed to protrude from the inner surface of portions 328 and 332. Member 392 provides a contour to the inner surface of the device so that the hand and wrist are retained in their natural position. Figure 16 shows a cross-section of contouring member 392 as taken along 16-16 of Figure 14, wherein member 392 is affixed to the exposed surface of layer 554 by suitable means, such as glueing and the like. Member 392 comprises a resilient, energy-absorbing plastic foam material and can be either an open-cell or closed-cell plastic foam material.

Figure 15 shows the device of Figure 13 as worn around and on the left hand and wrist, wherein member 320 is bent so as to form a relatively rigid, generally cylindrical surface 321 around the hand and wrist. Member 320 is bent about the hand so that contoured portion 328 abuts the top of the hand, contoured portion 326 abuts the palm, contoured portion 322 abuts the top of the arm behind the wrist, and contoured portion 330 abuts the inner arm below the wrist. Contouring member 329 is positioned to abut the back of the wrist to support the wrist and provide the natural contour to the wrist. Contoured portion 334 is positioned for the thumb to provide a comfortable fit, and to provide, in conjunction with member 392, a natural contour to the wrist. The device is secured on the hand and wrist by passing retaining strap member 386 through buckle 380, strap member 388 through buckle 382, and strap member 390 through buckle 384.

The device of Figure 13, when worn as shown in Figure 15, provides a rigid, generally cylindrical surface 321 as member 320 is bent around the hand and wrist. The natural contour of the wrist follows contouring member 392 and contoured portion 334, whereby the wrist is held in its natural, comfortable position. The device as shown in Figure 13 is for the left hand of the wearer. A similar device can be made for the right hand of the wearer.

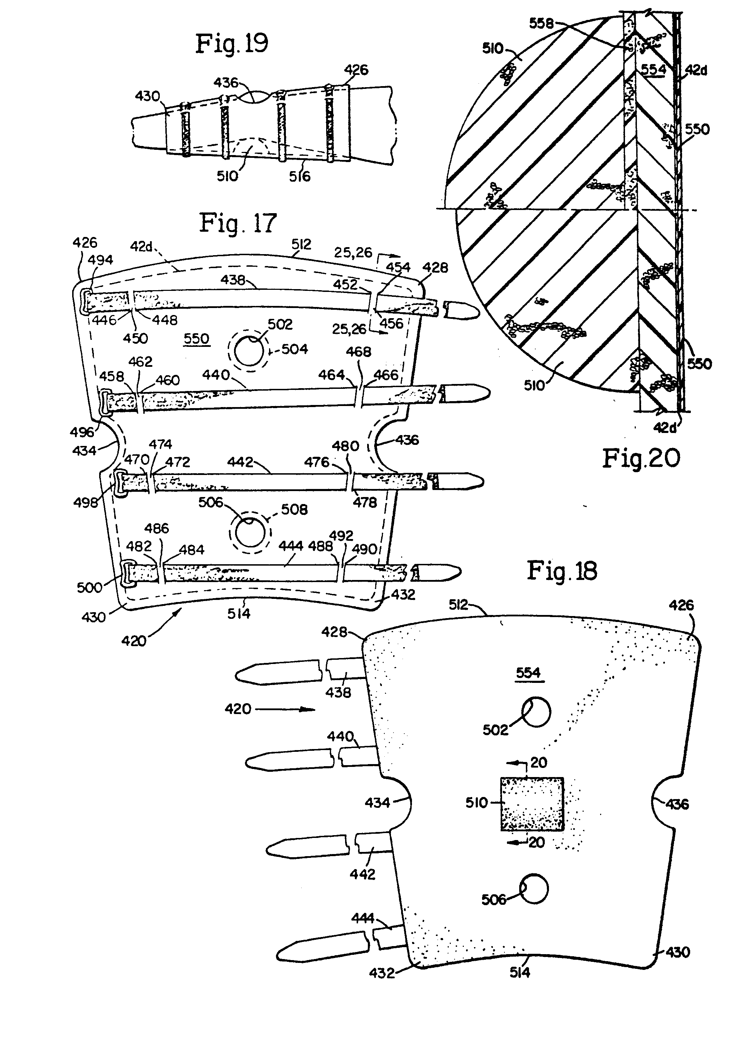

A fifth embodiment of the invention is shown in Figures 17-19 and the device, generally designated by the numeral 420, is designed for the protection and support of the leg portions or elbow and adjacent arm portions. Device 420 comprises a generally rectangular contoured shape having the sandwich-like construction of the invention, described above, and comprises contoured portions 426, 428, 430, 432, 434 and 436, ventilation openings 502 and 506, slits 446, 448, 452, 454 which form retaining loop members 450 and 456 for retaining strap member 438, slits 458, 460, 464 and 466 which form retaining loop members 462 and 468 for retaining strap member 440, slits 470, 472, 476, 478 which form retaining loop members 474 and 480 for retaining strap member 442 and slits 482, 484, 488 and 490 which form retaining loop members 486 and 492 for retaining strap member 444.

Embodiment 420 comprises a resilient plastic foam material layer 554, bendable stiffening material and outer covering material 550. The inward displacement of the bendable stiffening material 42d from the peripheral mating edges of the plastic foam material in layer 554 and outer covering material in layer 550 is shown in Figure 17. In addition, bendable stiffening material 42d is inwardly displaced from the edge of holes 502 and 506, as shown in Figure 17 by dotted lines 504 and 508. The stiffening material 42d is retained in its interposed position by securing the mating edges of plastic foam material in layer 554 and outer covering material in layer 550 by suitable means described above.

Figure 18 shows an inner planar view of the device of Figure 17 wherein a retaining, semi-cylindrical contouring member 510 is shown attached to the mid section of the surface of plastic foam material in layer 554 disposed to protrude from the surface.
Member 510 provides a contour to the inner surface of
the device to provide support to the body part so as to
provide a natural contour thereto.   Member 510
comprises a resilient, energy-absorbing plastic foam
material of either an open-cell or closed-cell structure,
and is secured to the surface by any suitable means.

Figure 19 shows the device of Figure 17 as it can be
worn on the knee and adjacent leg portions or on the
elbow and adjacent arm segments.   Figure 19 as
subsequently discussed, applies to the knee and adjacent
leg segments, however, it is understood that the device
as described can be worn in the same manner on the
elbow and adjacent arm portions.   Member 420 is worn
by bending it around the leg to form a rigid, generally
cylindrical surface 516.   The leg is thereby
substantially immobilised by the rigid support provided
by the bendable stiffening material 42d.   As shown in
Figure 19, member 420 is bent around the leg to
position contouring member 510 behind the knee to
provide support for and retain the natural contour of
the leg.   The knee cap portion of the knee extends
through the opening formed by abutting contoured
portions 434 and 436.   Contoured portion 428 (not shown
in Figure 19) and contoured portion 426 are positioned
so as to abut at the top of the leg above the knee,
and contoured portion 432 (not shown in Figure 19) and
contoured portion 430 are positioned so as to abut at
the shin below the knee.   The device is secured by
threading retaining strap members through their
respective buckles.   The end portions of the retaining
strap members are secured by self fastening material.

Figure 20 is a cross-section taken along line 20-20 of
Figure 18 and line 20-20 of Figure 14 and shows both
sandwich-like constructions of the invention shown in Figures 25 and 26 (which are described later) with contouring member 510 disposed on the surface of either layer 554 or on a layer 558. The member 510 can then be disposed on either surface in the embodiment 320 of Figures 13-15 and embodiment 420 of Figures 17-19.

A sixth embodiment of the invention is shown in Figures 21-24, wherein the device shown provides protection and support for the neck and head. The device, generally designated by the numeral 520, comprises a generally rectangular contoured shape having the sandwich-like construction of the invention comprising an outer covering material in layer 550, bendable stiffening material and resilient energy-absorbing material in layer 554.

Device 520 has contoured portions 528, 530,532 and 534, centre chin support member 524, and slits 536, 538, 540 and 542 which form loop members 540 and 544 respectively for a retaining strap member 536.

The bendable stiffening material 42e has the same overall contoured shape of member 520 but is slightly smaller in size and inwardly displaced as in the previous embodiments.

The peripheral edge of stiffening material 42e is shown by the dotted line in Figure 21 and is inwardly displaced from the peripheral edges of plastic foam material in layer 554 and outer covering material in layer 550.

Figure 22 shows the device of Figure 21, as worn around the neck and back of the head, with contoured portions 530, 534, 528 and 532 abutting at the back of the head and neck. Chin support member 524 is positioned under the chin to provide support for the chin and head when the device is worn. Retaining strap member 536 is fastened by the means of self fastening material. Contoured portions 533 and 536 with circular indentation 526 are positioned above the collar bone of the wearer and aid in retaining and limiting neck and head movements of the wearer whereas contoured portion 528 and 530 retain the back of the head.

Figure 23 shows in section the detail of the chin support member as 524 and Figure 24 shows a modification of the chin support member as 570. Support member 524 comprises an arched member, and support member 570 comprises a rolled cylindrical member upon which the chin rests. As shown in Figure 24 member 570 is formed by bending the centre support member until the end abuts the outer covering surface of layer 550 to which it is secured by suitable means.

Figure 25 shows construction of the layered material taken along line 25-25 of the various embodiments of the devices of the invention as shown in Figures 1, 5, 9, 13, 17, 21 and 27. The basic laminated, sandwich-like, construction comprises an inner layer of resilient energy-absorbing plastic closed - cell foam material 554, an adjacent bendable stiffening member 552, an outer layer of a pliable outer protective covering material 550 and a retaining strap member 556 with self fastening material 557 such as that sold under the Trade Name Velcro. The bendable stiffening member 552 is interposed between inner layer 554 of foam material and outer layer of 550 of covering material and is identified also in the previous embodiments by reference 42 and a relevant suffix.

Figure 26 shows a modification of the sandwich-like construction shown in Figure 25. The sandwich-like construction comprises an additional inner protective layer 558. Figure 26 shows the modified sandwich-like construction of the invention as taken along line 26-26 of the devices of Figures 1, 5, 9, 13, 17, 21 and 27 The modified layered arrangement comprises the outer layer 550 of a protective covering material, retaining strap member 556, self fastening material 557, layer 552 of a bendable stiffening material, inner layer 554 of resilient plastic foam material, and an additional inner protecting layer 558. Inner protecting layer 558 has the overall contoured shape of the particular embodiment of the protective device of the invention, and comprises a softer, flexible, resilient, energy-absorbing material with properties somewhat different from plastic foam material in layer 554. Material of layer 558 is preferably an open-cell plastic foam material which has a softer, cushioning characteristic than material 554. The use of the inner layer 558 aids in ventilation of the skin.

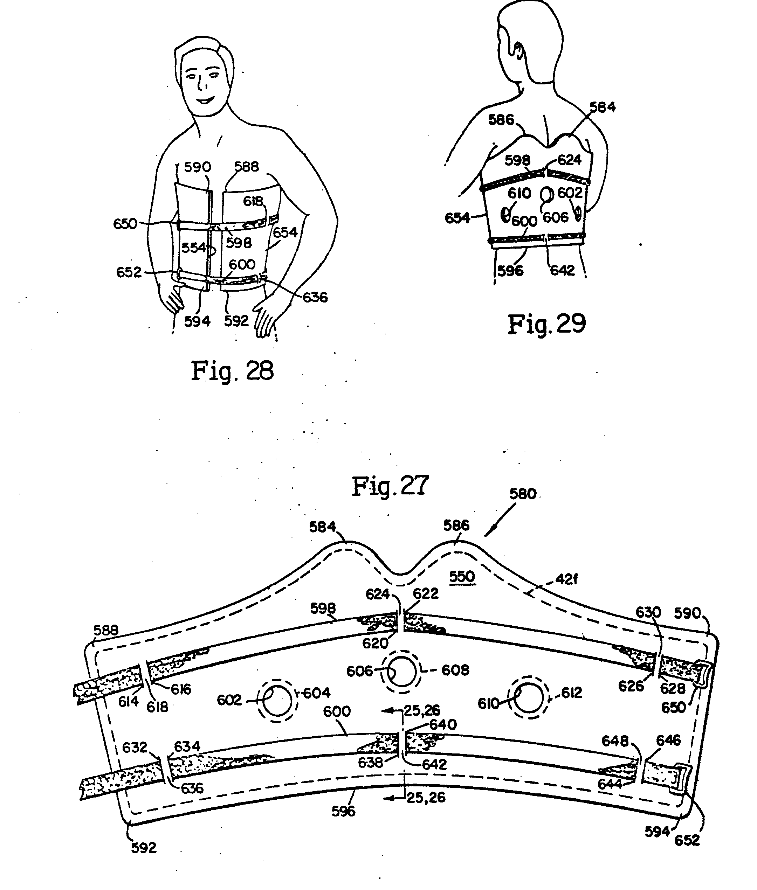

A seventh embodiment of the invention provides protection and support to the back, rib cage and abdomen, and the device of the invention is shown in Figures 27-29 and is generally designated by the numeral 580. Device 580 has a generally rectangular shape having the sandwich-like construction of the invention and comprises an outer covering material in layer 550, an inner, energy-absorbing material in layer 554, and a bendable stiffening material 42f in layer 552, with contoured portions 588, 590, 592, 594, 584, 586 and 596, slits 614, 616, 620, 622, 626 and 628 which form loop members 618, 624 and 630 respectively for retaining strap member 598, slits 632, 634, 638, 640, 644 and 646 which form loop members 636, 642 and 648 respectively for retaining strap member 600. Ventilation openings 602, 606 and 610 are provided to enable the passage of air to the body.

The bendable stiffening material 42f has the same overall contoured shape of the device but is slightly smaller in size as in previous embodiments whereby the peripheral edges of the stiffening material are inwardly displaced from the peripheral mating edges of layers 554 and 550 and is inwardly displaced from the edges of ventilation holes 602, 606, and 610, as shown in Figure 27 by dotted lines 604, 608 and 612.

Figures 28 and 29 show device 580 worn around the back, rib cage and abdomen as a relatively rigid, generally cylindrical shape 654. Contoured portions 588 and 590 abut the body in the region of the chest, contoured portions 592 and 594 abut the body in the region of the waist, contoured portions 584 and 586 abut the upper region of the waist, contoured portions 584 and 586 abut the upper region of the back to provide support to the back, and contoured portion 596 abuts the body in the region of the waist. The device is secured by threading retaining strap member 598 through buckle 650 and retaining strap member 600 through buckle 652. The ends of the retaining strap members are secured by the self fastening material.

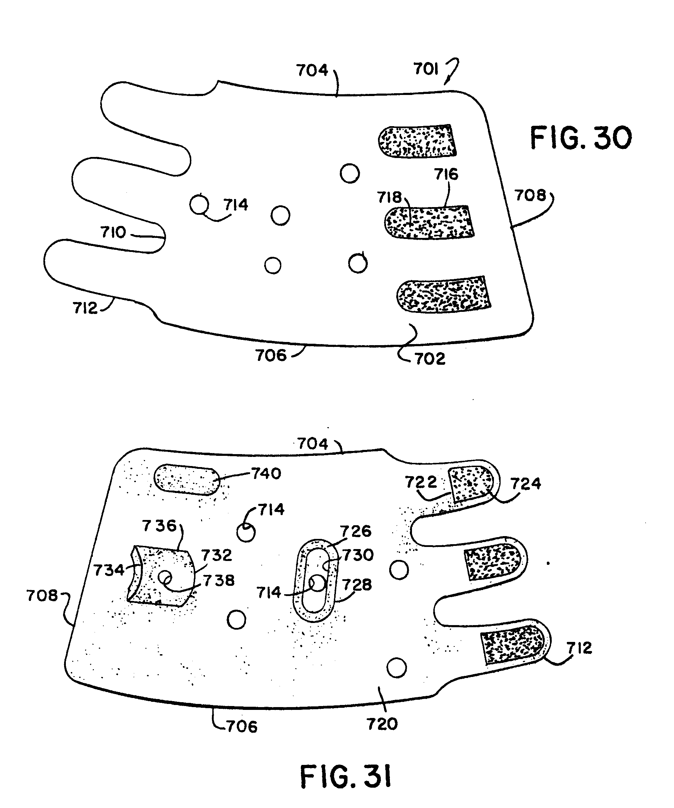

The cast is shown in Figure 31 from the position of a person sitting on a table who is about to place the cast around his left knee and outstretched left leg. Edge 704 represents the shorter distal edge of the cast and edge 706 represents the larger proxial edge of the cast. Edge 708 is placed along the outer side of the knee and the cast is bent around the knee, juxtaposing or superimposing edge 710 over edge 708 with flaps 712 overlying the outside of the cast and extending rearward. Ventilating openings 714 are provided throughout the cast. Parallel strips 716 of small hook-type fasteners 718 are provided on the outer surface 702 near edge 708.

The inside 720 of cast 701, as shown in Figure 31, has complementary patches 722 of pile-type fasteners 724 on flaps 712.

An oval annular shape insert 726 of closed cell foam material is glued to the inside 720 of the knee cast 701 in an area which overlies the knee cap.  In a preferred embodiment, the annular oval insert has an overall dimension of about 10 cms by 7.5 cms by 1.25 cms (4 inches by 3 inches by $\frac{1}{2}$ inch) in thickness with the central opening being approximately 2.5 cms by 5 cms (1 inch by 2 inches).  The oval shaped insert removes facial or lateral pressure from the knee cap and at the same time laterally stabilises the knee cap with respect to the cast.  A facial wall 728 of the insert 726 is approximately 2.5 cms (1 inch) across.

In one embodiment annular piece 726 is generally circular at its cut-out middle position is used as a knee backing pad 732.

Cylindrical insert 732 is positioned behind the knee. Insert 732 is formed as a segment of a cylinder and has circular lateral edges 734.  Edges 736 are bonded to the inner surface 720 of the knee cast.  A ventilation opening 738 is provided.

Cylindrical insert 732 preferably has a strip of hook-type fasteners on a flat outer face which engages a relatively long strip of pile-type fasteners on inner surface 720 of the cast to adjust for varying knee sizes.

The entire rear face of the anterior insert 732 may be bonded to the interior surface 720 of the knee cast or the central portion may be spaced from the inner surface 720.

An insert 740 positioned below the anterior of the knee fits beneath the calf muscle of the leg and aids the uniform contact of the cast with the leg around the knee area to immobilise the knee.

The knee cast described in Figures 30 and 31 may be constructed with straps such as shown in Figure 29 replacing the straps 724. Alternatively, the knee cast may be provided with short straps which loop through openings along edge 708.

The cast described with reference to Figures 30 and 31 may be dimensionally modified and used when it is necessary to hold the elbow in an unextended position.

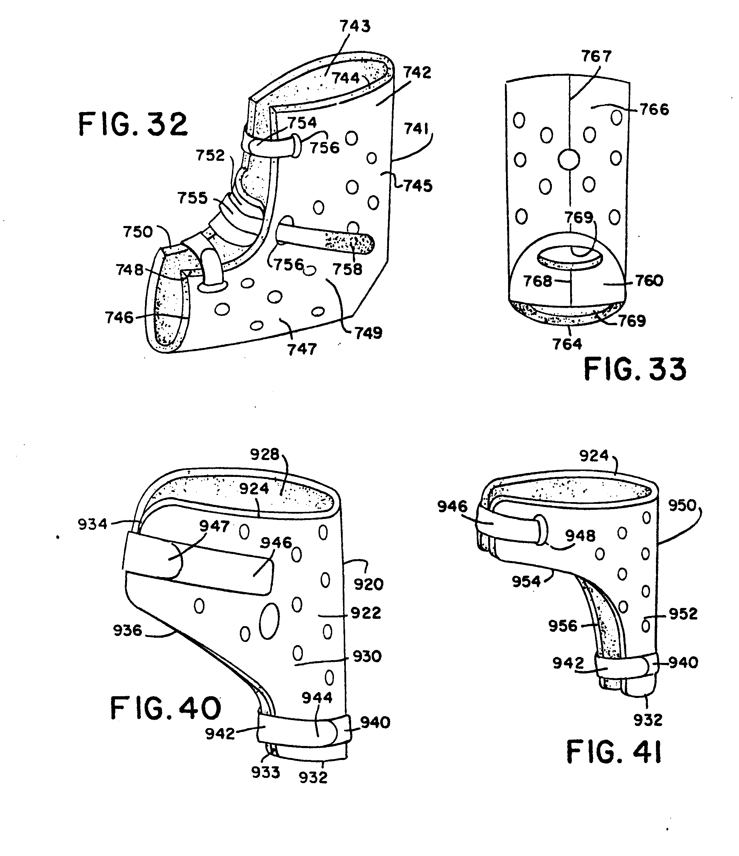

An elbow cast for a flexed elbow is described with reference to Figures 32 and 33. The elbow cast 741 has an exterior 742 which may be formed of a stiffening member or a stiffening member covered by a sheet material. The interior 743 is a closed cell foam material. Together, as is the case with other embodiments of the protective case, the closed cell foam material and the stiffening member cooperate so that when the foam material and the stiffening member are bent around one axis it is difficult if not impossible to flex the combined materials about any other non-concurrent or non-parallel axes.

The elbow cast has a generally cylindrical upper edge 744 and a generally cylindrical upper portion 745. A generally cylindrical lower edge 746 terminates a generally cylindrical lower portion 747. Frontal edges 748 and 750 connect the upper and lower edges 744 and 746 and extend over a central transitional area 749 where the cylindrical portions 745 and 747 join.

A flap 752 extends inward from edge 750 across the inside of the elbow. Straps 754, having pile type fasteners 755 across the major portion of their facial areas, are extended through fastening openings 756. Pulling on the straps through the opening and folding the straps back so that hook-type fasteners 758 near their distal ends engage the pipe type fastener 755 secures the cast on the elbow.

A soft portion 760 of closed cell material which is devoid of a stiffening member extends across the outer elbow.

Lower surface 764 of the elbow cast is formed without a seam. A rearward portion 766 of the upper portion 745 has a central seam 767 so that the elbow cast may be made of a single flat piece of material which is folded upward and joined along seam 767. Seam 768 may continue the seam 767 through the soft central member 760. Openings 769 are provided for ventilation and to prevent wrinkling of the sheet material as it is bent into cylindrical shape.

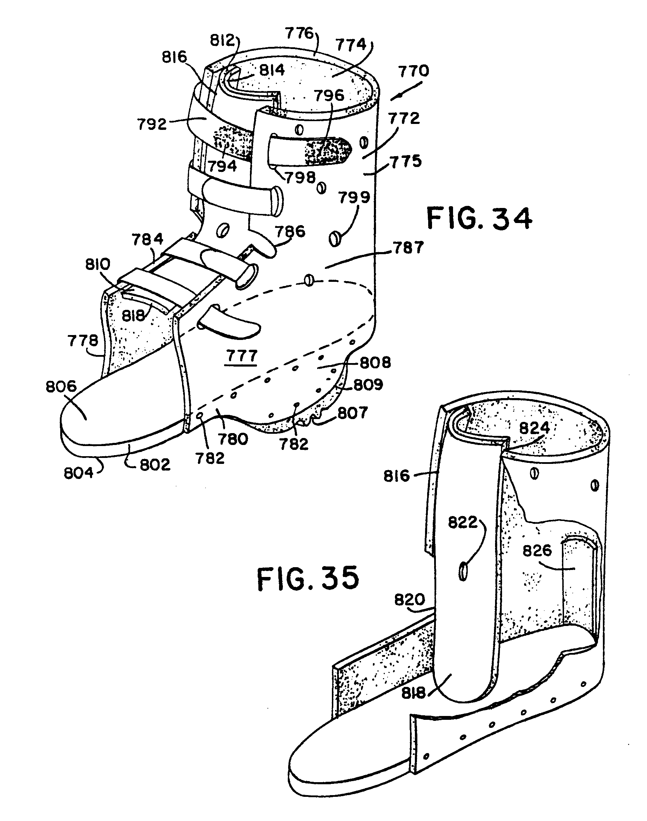

A foot cast 770 is shown with reference to Figures 34 and 35.

Foot cast 770 has a hard exterior surface 772 which may be formed of the outer surface of a stiffening member or a stiffening member covered by a sheet material. Inner surface 774 is constructed of a foam material. Together the closed cell foam material and the thin stiffening material form an arch like structure which, when bent around one axis, is difficult to bend around any other axes. The compressed foam in the arch like structure cooperates with the stressed stiffening member to form a rigid cast device.

The foot cast is made of a single piece of foam and stiffening material which is bent forwardly and secured to a sole. An upper portion 775 is formed generally as a cylinder with an upper circular edge 776. Lower side portions 777 terminate forwardly in edges 778. Lower edges 780 are nailed 782 and bonded or otherwise secured to a sole. Forward edges 784 of the foot cast are interrupted by notches 786 which permit bending of the upper portion 775 and the lower portion 777 about intersecting axes. The two portions are integrally joined along an imaginary line 787 which is common to both curves. Straps 792 have interfaces of proximal ends bonded to outer surface 772 of the foot cast. Pile type fasteners 794 along the outer middle portion of the straps cooperate with hook type fasteners 796 on the inner surfaces of distal ends of the strap. The distal ends of the straps are passed through holes 798 and are pulled tight before folding the distal ends back over the middle portions of the straps so that the hook type fasteners 796 engage the pile type fasteners 794. Ventilation holes 799 are provided throughout the cast.

The base 802 of the foot cast is made of a rigid construction which may be wood. A lower surface 804 may be coated with a non slip surface such as a patterned rubber sheet. An upper surface 806 of base 802 may be contoured to fit a sole of a foot or may have a contoured layer attached thereto. A rigid partially cylindrical block 808 with a rough lower surface 807 may be mounted beneath the base to facilitate walking on the cast. The outer and inner layers extend downward over opposite sides of block 808 to edges 809 and are bonded and nailed to the blocks to provide increased strength.

A tongue 810 is mounted inside the edge 784 of the cast. The tongue has an outer layer of closed cell foam material and an inner layer of open cell foam material for contacting the foot. The entire cast as well as other casts, may be constructed with open cell inner layers to provide ventilation throughout the cast near the skin of the wearer. The tongue has an upper portion 816 and a lower portion 818 and the straps 792 overlie the tongue.

As shown in Figure 35, the tongue is joined to an edge of the cast body in the upper portion 816. The lower edge 820 is free from the cast body. As shown in Figure 35, the lower end of the tongue may be rounded to promote comfort. One or more ventilation openings 822 are provided in the tongue. A central opening is significant additionally to provide flexure of the tongue between the upper and lower portions.

The free edge 824 of the tongue fits under the corresponding edge of the foot cast.

A cushion 826 is provided in the rear of the cast to cushion the heel portion and Achilles tendon area of a foot and ankle.

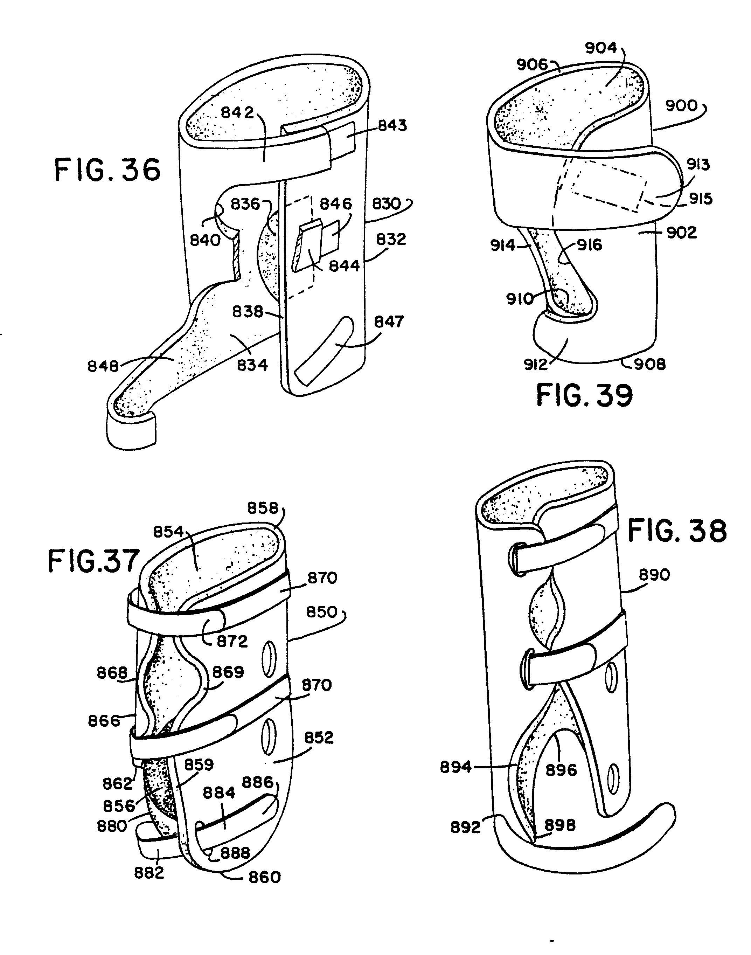

Wrists casts are shown in Figures 36, 37 and 38.

Wrists cast 830 shown in Figure 36 has an outer surface 832 which may comprise an outer surface of a stiffening material or a stiffening material covered by a sheet material. An inner surface 834 is constructed of closed-cell foam. A semi-cylindrical support 836 is bonded to the inner surface along edge 838 in an area adjacent the wrist. Opposite edge 840 has a plurality of straps 842, 844 and 848 which are integrally formed from the foam and skin material as flaps. Flap 842 has on its inner surface a strip of pile-type fasteners which engage a strip of hook-type fasteners 843 along the wrist edge of the wrist cast. Central flap 844 in a similar manner has pile-type fasteners which engage a strip 846 of hook-type fasteners in a central portion of the cast. Flap 848 has on its interior pile-type fasteners which engage sloped strip 847 of hook-type fasteners which is bonded to the outer surface 832 near the palm end of the cast.

In the cast shown in Figure 36, the cast is placed around the arm with the inside of the wrist carried on a semi-cylindrical insert 836. Flap 848 is folded around the palm betwen the thumb and the first finger and is secured to strip 847.

The insert 836 is formed of a closed-cell foam material. The insert in the entire cast may be lined with an open-celled foam material to aid circulation of air.

As shown in Figure 37, cast 850 has an outer surface 852 and an inner surface 854, similar to the other casts.

A semi-cylindrical insert 856 is bonded to the inner surface near lateral edge 859.

The arm opening edge 858 is substantially straight across. Edge 860 is curved to overlie the first joints of the fingers along the back of the hand and dips toward the wrist to expose the heel of the hand and the base of the palm adjacent the thumb along edge 862.

Side 866 of the cast underlies the wrist area and insert 856 overlies the back of the hand and the wrist area. Edges 868 and 869 are recessed to lie along the inside of the distal end of the arm. Straps 870 are bonded to the outer surface 852 and have ends 872 which extend through openings along the opposite edges and are folded upon themselves, securing the straps with hook and pile fasteners. Strap 882 is bonded to the outer surface of area 880 which overlies the back of the hand near the first joint of the little finger. The strap has hook fasteners 884 near its distal end 886 so that when the strap is pulled tight through opening 888 and folded upon itself, the hook fasteners cooperate with pile-type fasteners on the outer surface of the remainder of the strap.

The wrist cast 890 shown in Figure 38 is constructed similarly to the wrist cast 850 shown in Figure 37. The most significant difference is found in the lower portion 892 of the cast which underlies the first joint areas of the fingers along the inside of the palm. Edge 894 exposes the thumb and edge 896 encircles the hand along the first joints of the fingers. Edge 898 terminates along the first joint of the index finger. Pulling the straps through the openings and securing them upon themselves immobilizes the hand and wrist.

Finger and thumb casts are described with reference to Figures 39, 40 and 41.

Cast 900 shown in Figure 39 has an outer surface and an inner surface constructed similarly to the outer and inner surfaces of the casts previously described. Outer surface 902 is constructed of a stiffening material or a stiffening material covered by a sheet material. Inner surface 904 is constructed of the closed-cell foam. When the two joined materials are bent around the first axis, the materials may not be bent around further axes. An upper edge 906 encircles a wrist area of a hand, and a lower edge 908 encircles the thumb or fingers of a wearer. Flap 910 on the lower edge extends beneath flap 912. Hook-type fasteners are mounted on the outer surface flap 910 and pile-type fasteners are mounted on the complementary inner surface of flap 912.

The major flap 913 extends around the base of the palm, leaving the palm exposed. The flap 913 has inward facing pile-type fasteners which engage hooks on a slanted strip of hook fasteners shown in hidden lines 915.

The view in Figure 39 is taken from the direction of the thumb-side of the hand with the flap 912 being shown in its open position. Edge 914 extends downward along the thumb on the palm-side of the hand, and flap 913 extends around the palm-side of the hand along the shield of the hand to expose the entire palm such as for use during sports. Edge 19 extends down between the thumb and first finger across the back of the hand. With this cast the thumb is supported against shock and outward movement while substantially all the hand remains free for use.

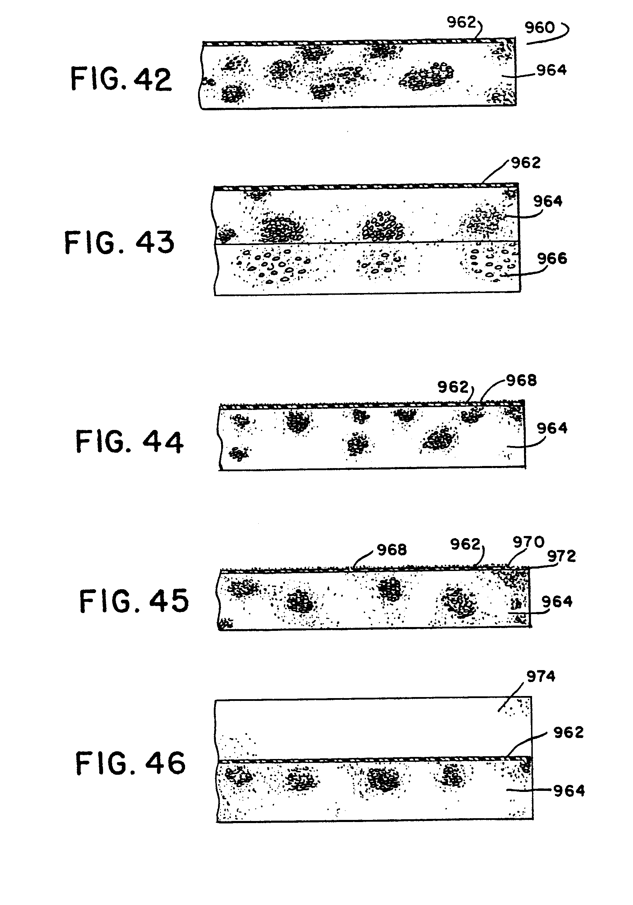

Figure 40 shows another embodiment of a thumb immobilising cast 920. The outer surface 922 is formed of a stiffening member which is bonded directly to a closed-cell foam material 928. The upper edge 924 extends around the wrist of the wearer, covering the heel of the hand and the opposite back of the hand with the major portion of the cast. The smaller portion of the cast 930 fits around the outside of the thumb of the wearer so that the lower edge lies in the area of the outer joint of the thumb. The inner edge 933 of the thumb portion 930 lies along the thumb. Edges 934 lie along the outer edge of the hand, and edges 936 diagonally overlie the palm of the hand and the back of the hand. A strip of hook fasteners 940 is mounted around the outer surface of the thumb portion. A separate strip 942 of inward facing pile fasteners is attached first at one end of 944 to the hook strip 940. Then strip 942 is pulled tight and the opposite end of the strip 942 is attached to the hook strip 940. Strap 946 having pile type fasteners is bonded to the outer surface 922. Free end 947 is passed through an opening similar to opening 948 shown in Figure 41 and the free end is pulled tight and the hooks on its inner surface are pressed on to the piles on the outer surface of strap 946.

A similar sports-type thumb supporting cast 950 is shown in Figure 41. As in cast 920, the thumb is immobilised with respect to the hand so that the thumb must move with the hand. The thumb covering portion 952 is elongated with respect to the base portion. Edges 954 expose the heel portion of the hand so that the entire palm of the hand is exposed such as for contact with sports equipment. Edge 956 lie along the side of the thumb, leaving the inside surface of the thumb exposed for contact sensation.

As shown in Figure 42 the basic construction of protective cast 960 of the present invention takes the form of an exoskeleton. Stiffening material 962 is placed as far outward as possible and closed-cell foam material 964 is bonded entirely along the inner surface of the stiffening member 962. When opposite ends of the structure 960 are bent downward around an axis extending perpendicularly to the drawing for example, the closed-cell foam material is placed under a compressive stress which tends to push the stiffening member outward into a desired uniform arcuate shape without irregular formations or abrupt changes. The stiffening member and the closed-cell foam material thus cooperate to readily permit bending about one axis, but once bent to prevent bending about any other axis thereby forming a rigid cast about a body member or part, limb, or extremity.

In some cases as shown in Figure 43 it is desirable to add an inner layer 966 of open-cell foam material which evenly distributes pressure and increases ventilation across the inner skin-contacting surface of the cast.

As shown in Figure 44, in some embodiments of the invention it is desirable to place a decorative and protective outer coating 968 on the outer surface of stiffening element 962. In Figure 45, the terminating of the stiffening member 962 short of the outward termination of the closed-cell foam material 964 is shown. The outer coating 968 extends over the edge 970 of the stiffening material 962 and tightly bonds to the exposed outward edge area 972 of the closed-cell foam material 964.

As shown in Figure 46, when the cast is constructed for use in situations in which blows on the outer surface of the cast might be expected, such as for use in sporting activities, a closed-cell foam material 974 is bonded to the outer surface of stiffening material 962. The stretching of the closed-cell material aids in the rigidity of the cast while at the same time protecting the wearer and those coming in contact with the cast from injury due to contact or shock.

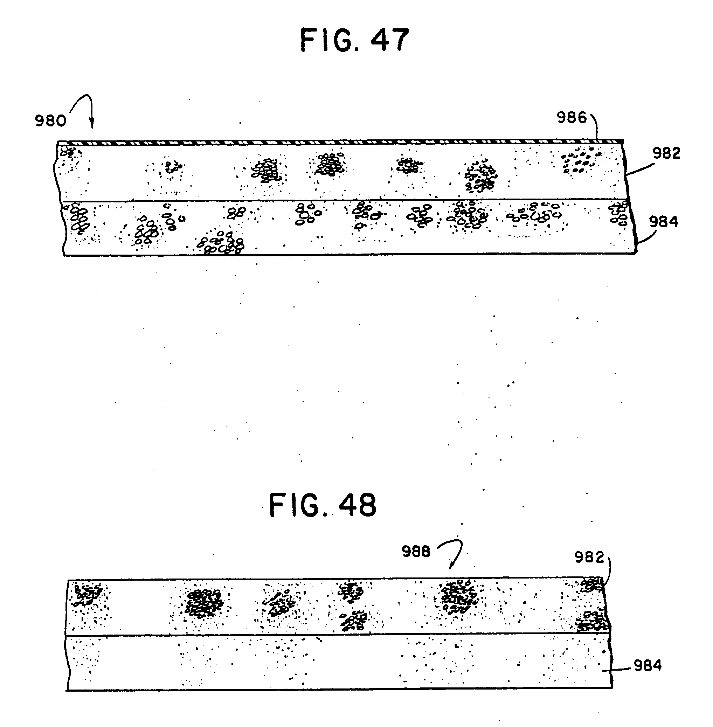

In Figure 47 a preferred light weight embodiment 980 of the invention is shown.   The embodiment 980 employs a bendable expanded material 982 which preferably has no memory and stays in its bended shape.   In one embodiment an expanded material 982, which may comprise a foam material which is preferably a closed-cell foam material, has an open cell foam material 984 secured to its inner side.   A tough flexible layer 986 which is freely flexible and bendable in all directions is bonded to the outside of the outer material 982.   In an embodiment 988 shown in Figure 48, the tough flexible leather-like outer layer 986 is omitted.   In the latter case, openings in the layers for receiving straps are reinforced to provide long wearing characteristics and to prevent creep or size change of the openings.

The expanded outer layer 982 becomes substantially rigid when bent into a generally cylindrical form and resists bending in any other direction.   The inner layer 984 of open cell material provides air circulation and comfort.

The outer layer 986 when used in the embodiment shown in Figure 47 tends to stretch and tends to press outer expanded layer 982 when the device is bent in cylindrical shape which further aids the rigidity of the protective cast.   Tough, thin, flexible outer surface 986 also promotes cleanliness of the cast and adds long wearing characteristics.

In all cases, the casts are constructed with ventilating openings which extend through the closed-cell foam material and through the stiffening material.   No loss of rigidity is experienced by using a large number of such openings.

The preferred closings for the casts are four in number. The most desirable closing, which is most effective and uniquely permits closure using one hand, is a short strap bonded to the outer surface of the protective cast and having pile-type fasteners along a major portion of the outer surface of the strap. A short end portion of the strap has hook-type fasteners upon its same outer surface in continuation of the pile surface. The free end of the strap is placed through an opening through the foam material and stiffening member along an opposite edge of the protective cast. Then the person pulls the free end toward the fixed end of the strap. This permits a person to pull opposite edges of the cast together and close the cast with one hand while at the same time doubling the force of pulling due to the mechanical advantage that the strap provides. Other preferred forms of closure are the bonding of hook patches on external surfaces of the cast and bonding pile material on internal surfaces of flaps extended from the cast, which flaps usually are devoid of stiffening material.

Another preferred form of closure is by the use of straps which extend completely around the cast and are secured by slits forming loops in the outer covering material and which straps have rings at one end for receiving an opposite free end and folding the free end back upon itself with hooks on the free end contacting piles on remainders of the straps.

The devices of the invention described above serve a dual purpose in protecting various parts of the body. The devices can be worn to protect the body from injury while engaging in contact sports and the like. The devices can also be worn on an injured part of the body as a temporary or permanent splint during healing thereof. The devices are particularly useful as temporary splints because they can be easily and
quickly placed and tightly retained around a body part
to maintain the body part, particularly the joints, in
an immobilised and unmovable condition.

The devices of the invention provide the desired
protection by virtue of the cylindrical form of the
device as it is worn on the body part.   The bendable
material being impact resistant serves to protect the
body part from blows and/or shocks while at the same
time maintaining the body part in a rigid immobilised
condition.   The recessed periphery design of the
bendable material is for the purpose of preventing the
peripheral edge of the bendable material from
contacting and possibly injuring the body part.   Any
contact of the body part with the peripheral edge of
the device is absorbed by the secured peripheral edges
of the energy-absorbing materials in the inner and
outer layers.

Claims:

1.  A protective device (30) for parts of the body characterized by comprising a sheet of energy absorbing cellular material (554) having a contoured edge (32, 34, 36, 38), the sheet being bendable to fit around a selected part of the body, and fastening means (59, 90; 107, 78; 111, 86) for securing the device (30) around the said selected part.

2.  A protective device as claimed in Claim 1 in which the energy absorbing cellular material (554) is a closed cell plastics foam material.

3.  A protective device as claimed in Claim 1 or Claim 2 and including an outer protective layer (550).

4.  A protective device as claimed in any preceding claim and including a layer of an impact-resistant stiffening material (552, 42) extending over the whole of one surface, or a part thereof, of the energy absorbing cellular material (554).

5.  A protective device as claimed in any preceding claim and including a layer of soft plastics material (966) bonded to an inner surface of the energy absorbing cellular material (964) so as to form, in use, a surface adjacent to the part of the body covered by the device which allows the circulation of air to be promoted across the surface of the said part of the body.

6.  A protective device as claimed in Claim 5 wherein the soft plastics material (966) is of an open-cell structure.

7.    A protective device as claimed in any preceding claim and including an outer layer (974) of a closed-cell plastics material as a shock absorbent layer.

8.    A protective device as claimed in any preceding claim characterised by the energy absorbing cellular material (982) being of a nature as to have no, or substantially no, memory when bent.

9.    A protective device as claimed in any preceding claim and including ventilation holes (154) extending through each layer (554, 552, 550).

10.    A protective device as claimed in any preceding claim wherein the fastening means includes straps integral with (712) or securable on (59, 107, 754) the device (701, 116, 741).

11.    A protective device as claimed in Claim 10 in which the straps (712) include self fastening elements (722, 724).

12.    A protective device as claimed in Claim 11 in which the elements comprise hook type fasteners (722) on one surface arranged to secure the surface to pile-type fasteners (724) on a second surface.

13.    A protective device as claimed in any preceding claim and including an insert (392) secured to the surface of the layer (554) which, in use, is adjacent the part of the body covered by the device in order to provide local support to a limited area of the body.

14.    A protective device as claimed in any preceding claim and including a plurality of parts (30,91) arranged to be disposed about a part of a body and to be relatively secured thereto by fastening means (59, 107,111) to give protection all around the said part.

Fig.3

Fig.2

Fig.1

Fig.4

Fig.6

Fig.7

Fig.5

Fig.8

Fig.10

Fig.11

Fig.12

Fig.9

0005615

4 / 13

Fig.15

Fig.13

Fig.16

Fig.14

Fig.19

Fig.17

Fig.20

Fig.18

Fig.22

531 530
524 534
536

Fig.23

524 550 554
536 42e

Fig.24

550
524 554
570 42e
536

Fig.21

528 529 524 520 530
25,26 536 531 42e
536 538 550 541 542
540 544
532 25,26 533 526 535 534
23

Fig.25

550 552 556 557
554

Fig.26

550 552 556 557
554
558

Fig. 28

Fig. 29

Fig. 27

FIG. 30

FIG. 31

FIG. 32

FIG. 33

FIG. 40

FIG. 41

FIG. 34

FIG. 35

FIG. 36

842
843
836
840
846
830
832
844
838
847
848
834

FIG. 39

906
904
900
913
915
902
914
916
910
912
908

FIG.37

858
854
870
850
872
868
869
870
866
852
862
859
856
880
884
886
882
888
860

FIG. 38

890
894
896
892
898

FIG. 42

FIG. 43

FIG. 44

FIG. 45

FIG. 46

FIG. 47

FIG. 48

European Patent Office

**EUROPEAN SEARCH REPORT**

Application number

**EP 79 30 0812**

| | DOCUMENTS CONSIDERED TO BE RELEVANT | | CLASSIFICATION OF THE APPLICATION (Int. Cl.²) |
|---|---|---|---|
| Category | Citation of document with indication, where appropriate, of relevant passages | Relevant to claim | |
| X | US - A - 2 785 407 (REEDER) <br><br> * column 1, line 54 to column 2, line 34; figures * <br><br> --- | 1,2,3, 4,7,8 | A 63 B 71/12 <br> A 61 F 5/04 |
| X | US - A - 3 146 461 (KAVANAGH) <br><br> * column 2, line 33 to column 3, line 33; figures * <br><br> --- | 1,2,3, 8,10, 14 | |
| X | US - A - 3 607 601 (MILAM) <br><br> * column 1, line 29 to column 2, line 58; figures * <br><br> --- | 1,2,5, 6,7,8 | TECHNICAL FIELDS SEARCHED (Int.Cl.²) <br><br> A 63 B <br> A 61 F |
| X | US - A - 3 975 015 (OWENS) <br><br> * column 2, line 48 to column 3, line 37; figures * <br><br> --- | 1,3,4, 5,6,10 | |
| | US - A - 2 553 612 (TAYLOR) <br><br> * column 1, line 52 to column 3, line 1; figures * <br><br> --- | 1,3,4, 5,8,9 | |
| | US - A - 3 921 222 (HOLLMAN) <br><br> * column 2, line 36 to column 3, line 42; figures * <br><br> --- | 1,3,4, 5,10, 11,12 <br><br> ./. | CATEGORY OF CITED DOCUMENTS <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |

The present search report has been drawn up for all claims

&: member of the same patent family, corresponding document

| Place of search | Date of completion of the search | Examiner |
|---|---|---|
| The Hague | 20-08-1979 | LEMERCIER |

EPO Form 1503.1  06.78

**European Patent Office**

**EUROPEAN SEARCH REPORT**

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | US − A − 2 266 886 (MacCOY)<br><br>* page 1, left−hand column, line 54 to page 2, left−hand column, line 30; figures *<br><br>──── | 1,3,4, 10 | |
| | US − A − 3 790 168 (HASHIMOTO)<br><br>* column 2, line 58 to column 3, line 56; figures *<br><br>──── | 1,3,4, 10,11, 12,13 | |
| | US − A − 3 909 847 (HOLT)<br><br>* column 1, line 54 to column 2, line 43; figures *<br><br>──── | 1,3,8, 14 | TECHNICAL FIELDS SEARCHED (Int. Cl.³) |
| | US − A − 3 158 871 (MORGAN)<br><br>* column 1, line 70 to column 3, line 37; figures *<br><br>──── | 1,3,10 14 | |
| P | US − A − 4 111 194 (COX)<br><br>* column 2, line 8 to column 3, line 55; figures *<br><br>─────────── | 1,3,4, 5,6,9, 10,11, 12,13 | |

EPO Form 1503.2  06.78